# EUROPÄISCHE PATENTSCHRIFT

(11) **EP 1 809 187 B1**
(45) Veröffentlichungstag und Bekanntmachung des Hinweises auf die Patenterteilung: **14.09.2011**
(21) Anmeldenummer: 05801888.8
(22) Anmeldetag: 05.11.2005
(51) Int. Cl.: A61B 17/22

(54) **MEHRDRAHTEINHEIT UND HERSTELLUNGSVERFAHREN HIERFÜR**
MULTIWIRE UNIT AND METHOD FOR PRODUCING THE SAME
UNITE MULTIFILAIRE ET PROCEDE DE FABRICATION

(30) Priorität: 08.11.2004 DE 102004055375
(43) Veröffentlichungstag der Anmeldung: 25.07.2007
(73) Patentinhaber: Epflex Feinwerktechnik GmbH, 72581 Dettingen/Ems (DE)
(72) Erfinder: UIHLEIN, Bernhard, 72581 Dettingen (DE)
(74) Vertreter: Patentanwälte Ruff, Wilhelm, Beier, Dauster & Partner
(86) Internationale Anmeldenummer: PCT/EP2005/011863
(87) Internationale Veröffentlichungsnummer: WO 2006/048314

(56) Entgegenhaltungen:
- WO-A-01/87168
- US-A- 5 499 981
- US-A1- 2002 068 954
- US-A1- 2004 026 942

## Beschreibung

Die Erfindung bezieht sich auf eine Mehrdrahteinheit nach dem Oberbegriff des Anspruchs 1 und auf ein Verfahren zur Herstellung einer solchen Mehrdrahteinheit.

Derartige Mehrdrahteinheiten werden beispielsweise als Drahtkorbeinheiten, die auch als Drahtkäfig- oder Fangkorbeinheiten bezeichnet werden können, für medizinische Instrumente zum Einfangen und Entfernen von Steinen oder anderen Ablagerungen aus menschlichen oder tierischen Gewebekanälen verwendet. Unter der Bezeichnung "Drahtkorb" ist dabei vorliegend zu verstehen, dass mehrere Drahtstücke um eine Längsachse der Drahtkorbeinheit herum mit Abstand voneinander in Umfangsrichtung im Wesentlichen axial verlaufend angeordnet sind, wobei sie sich in einem korbbildenden Zustand von einem vorderen Endbereich der Drahtkorbeinheit aus unter Bildung des Drahtkorbes zunächst radial nach außen aufweitend und dann wieder radial nach innen verengend erstrecken. Die Drahtstücke bestehen aus einem ausreichend elastischen, nicht notwendigerweise metallischen Material, wobei häufig superelastische Metalllegierungen verwendet werden.

Typischerweise sind die Drahtstücke in einem hinteren Endbereich durch eine Aufnahmehülse geführt, in die sie unter radialer Schrumpfung des Drahtkorbes eingezogen werden können. Im herausgezogenen, korbbildenden Zustand kann ein Stein oder dergleichen durch die Zwischenräume zwischen den Drahtstücken in den aufgeweiteten Drahtkorb hinein gelangen, wonach durch Einziehen der Drahtstücke in die Aufnahmehülse der Drahtkorb zusammengezogen und der Stein zwecks Abtransport festgehalten werden kann.

Medizinische instrumente dieser Art sind in verschiedenen Ausführengen beispielsweise als Endoskope in Gebrauch, wie als Steinfangkorb- und Ballonkathederinstrumente. Häufig ist bei diesen und anderen Anwendungen ein möglichst kurzer vorderer Endabschluss des Drahtkorbes erwünscht. Bei Konstruktionen, wie sie z.B. in der Patentschrift US 6.013.086 offenbart sind, werden die vorderen Drahtstückenden oder vordere Endschleifen von Drahtschlingen in einer zylindrischen Abschlusshülse gehalten, die folglich dem eigentlichen Drahtkorbbereich vorgelagert bleibt.

In der Offenlegungsschrift DE 197 22 429 A1 ist eine Vorrichtung zum Einfangen und/oder Zerkleinern von Gegenständen in Hohlorganen, insbesondere von Gallen- oder Nierensteinen, offenbart, die als gattungsgemäße Mehrdrahteinheit ausgeführt ist, d.h. sie beinhaltet mehrere Drahtstücke, die einstückig aus einem Rohrstück gebildet sind, dessen Rohrmantel in einem axialen Teilbereich durch mehrere, in Umfangsrichtung beabstandete Axialschlitze in die Drahtstücke aufgeteilt ist, wobei die Drahtstücke an einem vorderen Endbereich miteinander verbunden bleiben, indem die Axialschlitze dort unter Belassung einer vorderen Drahtverbindungsbereichs mit Abstand vor einem vorderen Rohrstirnende enden, und in einem vorgebbaren Funktionszustand der Mehrdrahteinheit anschließend an den Drahtverbindungsbereich einen gebogenen, körbchenbildenden Funktionszustand einnehmen. Bei dieser bekannten Mehrdrahteinheit enden die Axialschlitze mit relativ großem Abstand vor dem vorderen Rohrstirnende, so dass ein spitzenbildender vorderer Endstummel des Rohrstücks verbleibt, der den Drahtverbindungsbereich bildet. Der Abstand der Axialschlitze vom vorderen Rohrstirnende ist dabei deutlich größer als der Durchmesser des Rohrstücks und die Breite sowie die Dicke der Drahtstücke. Das z.B. aus Nickel-Titan-Draht bestehende Rohrstück mit den eingebrachten Drahtstücken dient als Zugstrang dieser Fangkorbvorrichtung. Das Einbringen der Axialschlitze erfolgt z.B. durch Laserstrahlschneiden. Das vordere Spitzenende der Fangvorrichtung kann mit einem halbkugelförmigen Abschlusselement und/oder einem röntgensichtbaren Einfassungsring versehen sein.

In ähnlicher Weise offenbart auch die Offenlegungsschrift US 2004/0026942 A1 eine gattungsgemäße Mehrdrahteinheit in Form eines Fangkorbinstruments, bei dem der Fangkorb aus einem Rohrstück unter Einbringen erster Axialschlitze und zusätzliches hälftiges Schlitzen jedes der durch die ersten Axialschlitze entstehenden Drahtstücke und anschließendes Aufbiegen der Drahtstücke gebildet ist. Auch bei diesem Fangkorbinstrument verbleibt am vorderen Rohrstückende ein spitzenbildender vorderer Endstummel des Rohrstücks, der je nach Bedarf relativ lang z.B. zum Aufsetzen einer Endhülse oder auch relativ kurz gehalten werden kann. Das Fangkorbinstrument ist mit einem Hohlkanal versehen, um einen Führungsdraht durchführen zu können.

Demgegenüber sind bereits sogenannte spitzenlose ("tipless") Drahtkorbeinheiten vorgeschlagen worden. So sind in der Offenlegungsschrift WO 98/36694 A1 Drahtkorbeinheiten beschrieben, bei denen die korbbildenden Drahtschleifen am Drahtkorbvorderende unter Bildung engerer Schlingen miteinander lose und damit gelenkig verknüpft sind. In der Patentschrift DE 101 17 836 C1 wird vorgeschlagen, die Drahtstücke am Vorderende gelenkig an einem Fixierkörper zu halten, der so gestaltet sein kann, dass sich im korbbildenden Zustand ein praktisch spitzenloses Vorderende der Drahtkorbeinheit ergibt.

Des weiteren sind Mehrdrahteinheiten in medizinischen Instrumenten auch als Drahtfilter, wie Koronarfilter, zum Abfangen von Ablagerungen oder Verklumpungen in Gewebekanälen gebräuchlich, z.B. zur Verhinderung von Embolien.

Der Erfindung liegt als technisches Problem die Bereitstellung einer Mehrdrahteinheit der eingangs genannten Art, die sich mit vergleichsweise geringem Aufwand spitzenlos realisieren lässt, und eines zugehörigen Herstellungsverfahrens zugrunde.

Die Erfindung löst dieses Problem durch die Bereitstellung einer Drahtkorbeinheit mit den Merkmalen des Anspruchs 1 und eines Herstellungsverfahrens mit den Merkmalen des Anspruchs 11.

Die erfindungsgemäße Mehrdrahteinheit lässt sich einstückig aus einem Rohrstück bilden, indem in dessen Rohrmantel mehrere, in Umfangsrichtung beabstandete Axialschlitze eingebracht werden, die unter Belassung eines Drahtverbindungsbereichs mit Abstand vor einem Rohrstirnende enden. Dadurch wird der Rohrmantels in mehrere Drahtstücken aufgeteilt, die in einem an den Drahtverbindungsbereich anschließenden Abschnitt in die für einen entsprechenden Funktionszustand der Mehrdrahteinheit gewünschte Gestalt gebogen werden, wobei sich charakteristischerweise auch der Drahtverbindungsbereich verformt, so dass ein im Wesentlichen spitzenloser Endabschluss gebildet ist.

Die Erfindung ermöglicht so die Herstellung einer spitzenlosen Mehrdrahteinheit aus nur einem einzigen Teil, d.h. dem Rohrstück. Der vordere Drahtverbindungsbereich, über den die Drahtstücke am vorderen Endbereich zusammenhängend miteinander verbunden bleiben, lässt sich je nach Bedarf so gestalten, dass sich bei entsprechendem Auf- bzw. Umbiegen der Drahtstücke z.B. ein praktisch spitzenloser Drahtkorb oder ein praktisch spitzenloser Drahtfilter mit gewünschter maximaler Filterdurchlassweite ergibt. Dazu erstrecken sich die Axialschlitze bis zu einem relativ geringen Abstand vom vorderen Rohrstirnende, so dass sich beim Auf- bzw. Umbiegen der Drahtstücke auch der Drahtverbindungsbereich umformt und kein merklicher axialer Rohrstückendstummel verbleibt, d.h. die Mehrdrahteinheit endet in ihrem Funktionszustand im wesentlichen spitzenlos, indem sich auch der Drahtverbindungsbereich selbst unter Materialverformung biegt.

Mit der Bezeichnung "spitzenlos" ist vorliegend, wie der Fachmann versteht, eine vordere Endabschlussgestaltung der Mehrdrahteinheit zu verstehen, die entweder gar keinen axialen, auf das zugrundeliegende Rohrstück zurückgehenden Endstummel oder einen lediglich sehr geringfügigen Endstummel aufweist, dessen Dimensionierung und insbesondere axiale Länge im Vergleich zu den Abmessungen des anschließenden, von den Drahtstücken gebildeten Funktionsteils, wie eines Drahtkorbs oder eines Drahtfilters, vernachlässigbar klein ist, z.B. nur eine axiale Länge aufweist, die um eine oder mehrere Größenordnungen kleiner als der maximale Durchmesser eines anschließenden Drahtkorbes ist. Insbesondere bedeutet der Begriff "spitzenlos" oder "im wesentlichen spitzenlos", dass kein verbleibender axialer Endstummel abrupt von der anschließenden, von den Drahtstücken gebildeten Funktionseinheit nach vorn vorsteht, sondern die Mehrdrahteinheit schon von ihrem Vorderende aus in einem gegenüber dem Rohrstückrohling aufgebogenen Verlauf vom Drahtverbindungsbereich in den gebogenen Verlauf der Drahtstücke im Funktionsteil übergeht.

In einer vorteilhaften Ausgestaltung der Erfindung besteht das Rohrstück und damit die Mehrdrahteinheit aus einem superelastischen Material. Solche Materialien haben in aller Regel auch ein Formgedächtnis. Letzteres lässt sich dazu nutzen, den Funktionszustand der Drahtstücke als den formstabilen Zustand zu wählen, so dass die Drahtstücke von selbst ihren Funktionszustand einnehmen, wenn sie nicht durch äußere Kräfte, z.B. durch Einschieben in eine Aufnahmehülse, daran gehindert bzw. in einen anderen Zustand gebracht werden.

In einer Weiterbildung der Erfindung werden die Drahtstücke am Drahtverbindungsbereich um mindestens ca. 60°, bei Bedarf um ca. 90° oder mehr, relativ zu ihrer axialen Rohrmantellage in den Funktionszustand gebogen. So erlaubt z.B. eine Aufbiegung um ca. 90° die Realisierung eines vollständig stumpf endenden Drahtkorbes, der sich am Vorderende mit allenfalls geringer Axialkomponente im wesentlichen radial nach außen weitet, wie dies vor allem für medizinische Instrumente häufig gewünscht ist, oder eines ebenfalls in medizinischen Instrumenten verwendbaren Drahtfilters. Zur Bereitstellung entsprechender Mehrdrahteinheiten kann auch ein Umbiegen der Drahtstücke um mehr als 90° vorgesehen sein, d.h. praktisch ein wenigstens bereichsweises Umstülpen des in die Drahtstücke aufgeteilten Rohrstücks.

Eine vorteilhafte Biegbarkeit des Drahtverbindungsbereichs, um den Funktionszustand der Mehrdrahteinheit zu bilden, ist in einer Ausgestaltung der Erfindung dadurch gegeben, das der axiale Abstand der Axialschlitze vom vorderen Rohrstirnende kleiner als ein Außendurchmesser und/oder ein Innendurchmesser und/oder etwa gleich groß wie oder kleiner als eine Wanddicke des Rohrstücks und/oder etwa gleich groß wie oder kleiner als eine Breite der Drahtstücke gewählt ist. Je nach konkreter sonstiger Gestaltung des Drahtverbindungsbereichs ermöglicht dies ein Umformen des endseitigen Drahtverbindungsbereichs derart, dass kein merklicher axialer Endstummel des Rohrstücks verbleibt, d.h. es wird ein spitzenloser Endabschluss der Mehrdrahteinheit in einer gewünschten Gestaltung des Drahtverbindungsbereichs erzielt.

In einer vorteilhaften Ausgestaltung der Erfindung werden in den Rohrmantel von der Rohrstirnseite her oder dieser benachbart eine oder mehrere schlitzförmige Ausnehmungen eingebracht, die sich in Rohrumfangsrichtung an Winkellagen zwischen den Winkellagen der Axialschlitze befinden. Mit diesen schlitzförmigen Ausnehmungen lässt sich das Biegeverhalten der Drahtstücke im Drahtverbindungsbereich und die Gestalt des Drahtverbindungsbereichs im Funktionszustand der Mehrdrahteinheit in jeweils gewünschter Weise beeinflussen.

In einer vorteilhaften Weiterbildung erstrecken sich die schlitzförmigen Ausnehmungen axial bis in den Bereich zwischen die Axialschlitze. In weiterer Ausgestaltung sind die Axialschlitze in ihrem mit den schlitzförmigen Ausnehmungen überlappenden Bereich verjüngt, d.h. sie weisen dort eine geringere Breite auf als in ihrem hinteren Teil außerhalb dieses Überlappungsbereichs. Vorteilhafte Gestaltungen der Axialschlitze und der schlitzförmigen Ausnehmungen im überlappenden Axialbereich sind beispielsweise solche mit im wesentlichen gleichmäßiger Breite der Axialschlitze und der schlitzförmigen Ausnehmungen oder solche mit sich gegensätzlich keilförmig verjüngenden Axialschlitzen und schlitzförmigen Ausnehmungen. Eine weitere vorteilhafte Gestaltungsvariante sieht mehrere Reihen von in Umfangsrichtung aufeinander folgenden schlitzförmigen Ausnehmungen vor, wobei diese Reihen in Axia!r'chtung versetzt sind und sich axial überlappen oder nicht überlappen.

In einer weiteren vorteilhaften Ausgestaltung der Erfindung ist das Rohrstirnende mit einer äußeren, umlaufenden Profilierung versehen, z.B. einem wellenförmigen Profil. Auch mit dieser Maßnahme kann die Gestalt des Drahtverbindungsbereichs im Funktionszustand der Mehrdrahteinheit ebenso wie das Biegeverhalten der Drahtstücke in diesem Bereich in einer gewünschten Weise beeinflusst werden.

Vorteilhafte Ausführungsformen der Erfindung sind in den Zeichnungen dargestellt und werden nachfolgend beschrieben. Hierbei zeigen:
- Fig. 1: eine Seitenansicht auf einen Vorderbereich eines Rohrstücks mit eingebrachten Axialschlitzen zur Herstellung einer einstü- ckigen Drahtkorbeinheit mit vier Drahtstücken,
- Fig. 2: eine Querschnittansicht längs der Linie II-II von Fig. 1,
- Fig. 3: eine Perspektivansicht auf den Stirnendbereich des Rohr- stücks von Fig. 1,
- Fig. 4 bis 6: Seitenansichten des vorderen Endbereichs des Rohrstücks von Fig. 1 in einer Anfangslage, einer Zwischenlage bzw. ei- ner Endlage beim Aufbiegen der durch die Axialschlitze aus dem Rohrstück von Fig. 1 erzeugten Drahtstücke in einen korbbildenden Zustand,
- Fig. 7 bis 9: zu den Fig. 4 bis 6 korrespondierende Längsschnittansich- ten des vorderen Endbereichs des Rohrstücks von Fig. 1,
- Fig. 10 bis 12: zu den Fig. 7 bis 9 korrespondierende Detailansichten eines ausschnittweisen Bereichs X von Fig. 7,
- Fig. 13: eine Draufsicht auf die Vorderseite der gemäß den Fig. 1 bis 12 gebildeten Drahtkorbeinheit,
- Fig. 14: eine Seitenansicht der Drahtkorbeinheit von Fig. 13,
- Fig. 15: eine Abwicklungsansicht des vorderen Endbereichs einer Va- riante des Rohrstücks von Fig. 1 mit wellenförmig profilierter Rohrstirnseite,
- Fig. 16: eine Draufsicht auf die Vorderseite der aus dem Rohrstück gemäß Fig. 15 erhaltenen Drahtkorbeinheit,
- Fig. 17: eine Perspektivansicht des vorderen Endbereichs des Rohr- stücks gemäß Fig. 15,
- Fig. 18: eine Abwicklungsansicht entsprechend Fig. 15 für eine Rohr- stückvariante mit nach außen offenen, axialen schlitzförmigen Ausnehmungen zwischen den und überlappend mit den Axi- alschlitzen, wobei die schlitzförmigen Ausnehmungen und die Axialschlitze im Überlappungsbereich eine jeweils gleichblei- bende Breite aufweisen,
- Fig. 19: eine Draufsicht entsprechend Fig. 16 auf die Vorderseite der aus dem Rohrstück von Fig. 18 gebildeten Drahtkorbeinheit,
- Fig. 20: eine Perspektivansicht entsprechend Fig. 17 für die Draht- korbeinheit von Fig. 19,
- Fig. 21: eine Abwicklungsansicht des vorderen Rohrendbereichs für eine Variante des Rohrstücks von Fig. 18 mit sechs Drahtstü- cken und gegensätzlich keilförmigen schlitzförmigen Aus- nehmungen und Axialschlitzen,
- Fig. 22: eine Draufsicht entsprechend Fig. 19 auf die Vorderseite der aus dem Rohrstück von Fig. 21 gebildeten Drahtkorbeinheit,
- Fig. 23: eine Abwicklungsansicht entsprechend Fig. 21 für eine Rohr- stückvariante mit nach außen geschlossenen schlitzförmigen Ausnehmungen,
- Fig. 24: eine Abwicklungsansicht entsprechend Fig. 23 für eine weite- re Variante mit nach außen geschlossenen schlitzförmigen Ausnehmungen,
- Fig. 25: eine Draufsicht entsprechend Fig. 22 auf die Vorderseite der aus dem Rohrstück von Fig. 24 gebildeten Drahtkorbeinheit,
- Fig. 26: eine Abwicklungsansicht entsprechend Fig. 24 für eine Rohr- stückvariante mit schlitzförmigen Ausnehmungen von gleich- bleibender Breite,
- Fig. 27: eine Draufsicht entsprechend Fig. 25 auf die Vorderseite der aus dem Rohrstück von Fig. 26 gebildeten Drahtkorbeinheit,
- Fig. 28: eine Abwicklungsansicht entsprechend Fig. 26 für eine weite- re Rohrstückvariante mit modifizierter Gestaltung der schlitz- förmigen Ausnehmungen und
- Fig. 29: eine Abwicklungsansicht entsprechend Fig. 21 für eine Rohr- stückvariante mit mehreren Reihen schlitzförmiger Ausneh- mungen.

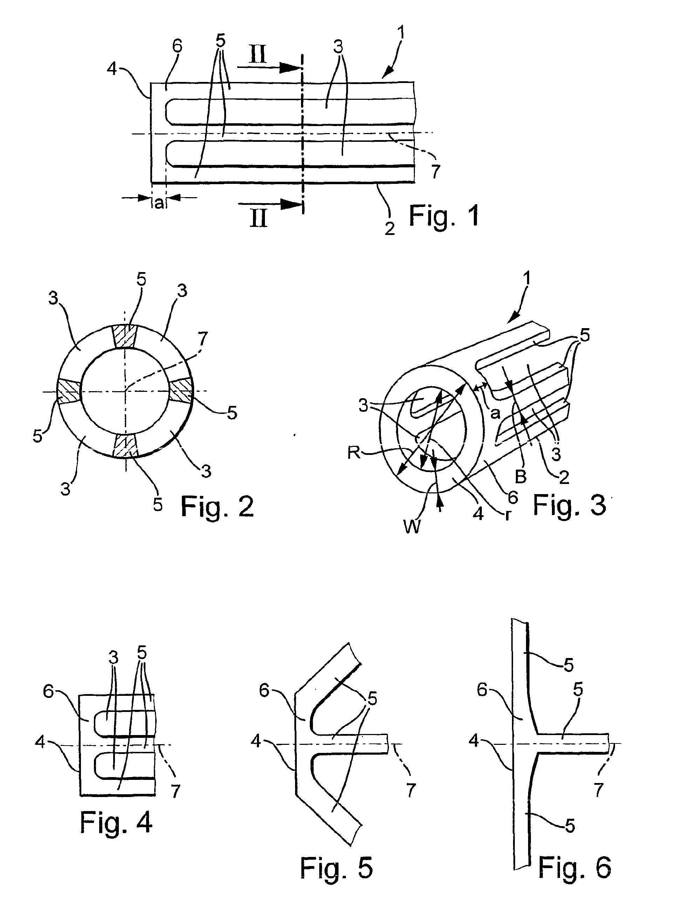

Fig. 1 zeigt den hier interessierenden, vorderen Teil eines hohlen Rohrstücks 1, das zur einstückigen Bildung einer Drahtkorbeinheit dient und z.B. aus einem superelastischen Material, insbesondere einem solchen mit Formgedächtnis, besteht, wie aus einer NiTi-Legierung, die auch unter dem Handelsnamen Nitinol bekannt ist.

Wie aus Fig. 1 in Verbindung mit den Fig. 2 und 3 ersichtlich, sind in einen Rohrmantel 2 des Rohrstücks 1 vier Axialschlitze 3 eingebracht, die in Rohrumfangsrichtung äquidistant voneinander beabstandet sind und in Axialrichtung mit vorgebbarem Abstand a vor einer zugehörigen Rohrstirnseite 4 enden. Durch die vier axial verlaufenden Schlitze 3 ist der Rohrmantel 2 in vier Drahtstücke 5 aufgeteilt, die in Rohrumfangsrichtung um jeweils 90° voneinander beabstandet sind. Da die Axialschlitze 3 nicht bis zur Rohrstirnseite 4 durchgehen, sondern mit dem besagten axialen Abstand a vor dieser enden, bildet dieser vordere, in Umfangsrichtung durchgängig verbleibende Rohrmantelabschnitt einen Drahtverbindungsbereich 6, über den die Drahtstücke 5 zusammenhängen, d.h. miteinander verbunden bleiben.

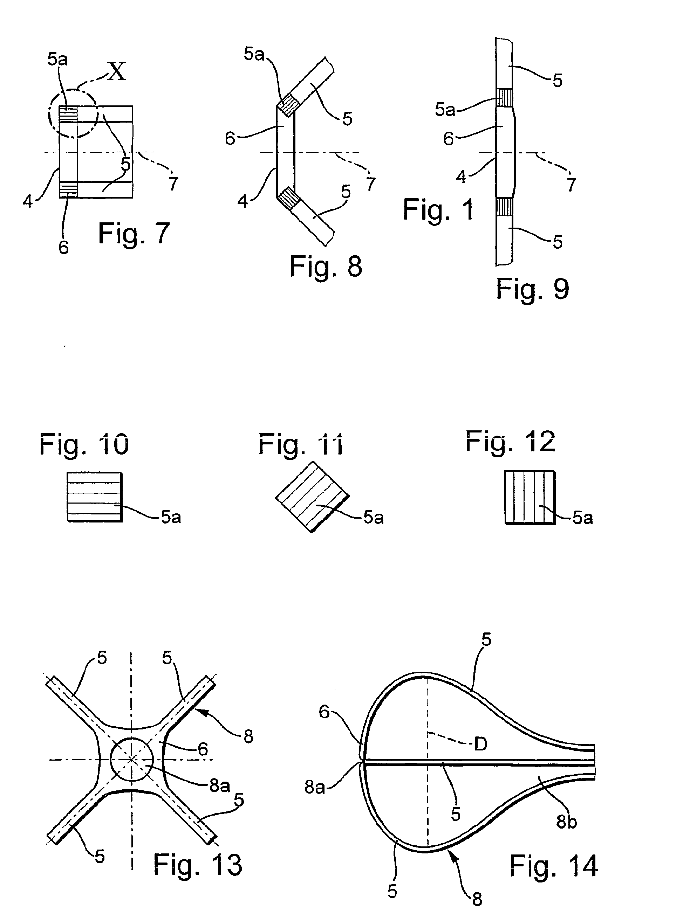

Aus dem dergestalt vorbereiteten Rohrstück 1 lässt sich eine Drahtkorbeinheit unter korbbildender Umformung der vier Drahtstücke 5 herstellen, wie dies in den Fig. 4 bis 14 veranschaulicht ist. Dazu werden die Drahtstücke 5 aus ihrer zur Rohrstücklängsachse 7 parallelen Lage herausgebogen und korbbildend umgeformt, wobei sie im distalen, vorderen Drahtverbindungsbereich 6 ebenso wie in einem nicht gezeigten, proximalen, hinteren Schaftbereich einteilig miteinander verbunden bleiben. Im Drahtverbindungsbereich 6 erfolgt hierbei eine Aufbiegung der Drahtstücke 5 um ca. 90°. Die Fig. 4 und 7 illustrieren den vorderen Rohrendhereich vor dem Biegevorgang, die Fig. 6 und 9 illustrieren diesen Rohrendbereich nach abgeschlossener Umbiegung, und die Fig. 5 und 8 illustrieren eine Zwischenposition nach ca. hälftiger Umbiegung. Die Fig. 10 bis 12 veranschaulichen den Biegeprozess anhand einer Vergrößerung des in Fig. 7 bezeichneten Ausschnitts X. Mit Streifenlinien ist in den Fig. 10 bis 12 die Materialorientierung während des Biegeprozesses in diesem Bereich symbolisiert. Dies zeigt, dass die Drahtstücke 5 beim Biegeprozess mit ihrem vorderen Endbereich 5a um ca. 90° von ihrer zur Rohrlängsachse 7 parallelen in eine dazu senkrechte Lage radial nach außen weisend umgebogen werden, wobei sich der Drahtverbindungsbereich 6 entsprechend nachgiebig verformt.

Die Fig. 13 und 14 zeigen die durch diesen Biegeprozess erzeugte Drahtkorbeinheit in einer Vorderansicht bzw. einer Seitenansicht. Wie daraus ersichtlich, sind in diesem korbbildenden Zustand die Drahtstücke 5 unter Bildung eines Drahtkorbes 8 gewünschter Form so umgebogen, dass sich ein vom Drahtverbindungsbereich 6 gebildetes, spitzenloses Korbvorderende 8a ergibt. Vom Korbvorderende 8a erstrecken sich die Drahtstücke 5 zunächst mit sehr geringer und dann mit zunehmender Axialkomponente radial nach außen, bevor sie sich ab einem maximalen Korbdurchmesser D wieder radial verengen und bis auf einen geringen Radialabstand am hinteren Korbende 8b zusammenlaufen.

Dieses Mitbiegen bzw. Mitumformen des Drahtverbindungsbereichs 6 beim Biegen der Drahtstücke 5 wird dadurch ermöglicht, dass die Axialschlitze 3 geeignet in den Rohrmantel 2 des Rohrstücks 1 eingebracht sind, insbesondere mit ausreichend geringem Abstand a zur vorderen Rohrstirnseite 4. Wie in Fig. 3 dargestellt, ist dieser axiale Abstand a insbesondere kleiner gewählt als ein Außendurchmesser R und kleiner als ein Innendurchmesser r des Rohrstücks 1. Wenngleich in Fig. 3 nicht maßstäblich wiedergegeben, ist der axiale Abstand a in vorteilhaften Ausführungsbeispielen um ein Mehrfaches kleiner als der Rohraußendurchmesser R und liegt beispielsweise etwa in der Größenordnung einer Wanddicke W des Rohrstücks 1 und/oder einer Breite B der Drahtstücke 5. Je nach Bedarf und Anwendungsfall kann der axiale Abstand a der Axialschlitze 3 vom vorderen Rohrstirnende 4 auch kleiner als die Rohrwanddicke W und/oder die Drahtstückbreite B sein.

In nicht gezeigter, üblicher Weise können die Drahtstücke 5 z.B. in einer Aufnahmehülse axial verschieblich gehalten sein. Durch Einziehen der elastischen Drahtstücke 5 in die Aufnahmehülse lässt sich der Drahtkorb 8 zunehmend verengen. Dabei kann vorgesehen sein, dass die Drahtstücke 5 vollständig in die Aufnahmehülse eingeschoben werden können. Beim Herausbewegen der Drahtstücke 5 aus der Aufnahmehülse nehmen die Drahtstücke 5 wieder ihren korbbildenden Zustand ein.

Mit diesen Funktionalitäten kann die Drahtkorbeinheit insbesondere als medizinisches Fangkorbinstrument verwendet werden. Dazu wird sie mit in die Aufnahmehülse eingeschobenen Drahtstücken 5 in einen Gewebekanal eingebracht, wonach die Drahtstücke 5 aus der Aufnahmehülse herausgeschoben werden und den aufgeweiteten Drahtkorb 8 bilden. Durch die Zwischenräume zwischen den Drahtstücken 5 können Steine oder andere Gewebeablagerungen in das Innere des Drahtkorbes 8 gelangen. Dann werden die Drahtstücke 5 wieder zurückgezogen, wodurch sich der Drahtkorb 8 verengt und die eingefangenen Steine bzw. Ablagerungen festhält, so dass sie anschließend aus dem Gewebekanal heraustransportiert werden können.

Die obige Beschreibung des Ausführungsbeispiels gemäß den Fig. 1 bis 14 macht deutlich, dass die Erfindung die Realisierung einer Drahtkorbeinheit mit sehr geringem Aufwand als einstückig aus einem Rohrstück gefertigten Bauteil ermöglicht, insbesondere auch in einer Ausführung mit spitzenlosem Drahtkorb. Die Dimensionierung der Drahtkorbeinheit, wie bezüglich Länge und Querschnitt der Drahtstücke, Durchmesser des Rohrstücks, Rohrmanteldicke und Drahtkorbdurchmesser, unterliegt keinen spezifischen Beschränkungen und kann sich daher frei nach den Anforderungen des jeweiligen Anwendungsfalls richten. So sind z.B. für die Verwendung in medizinischen Fangkorbinstrumenten Rohrdurchmesser zwischen etwa 0,5mm und etwa 2mm im allgemeinen von Vorteil, bei Bedarf sind jedoch auch beliebige andere Durchmesser des drahtkorbbildenden Rohrstücks möglich. Das Einbringen der Axialschlitze 3 in den Rohrmantel 2 kann durch ein beliebiges, hierfür gängiges Verfahren erfolgen, wie Laser- oder Wasserstrahlschneiden.

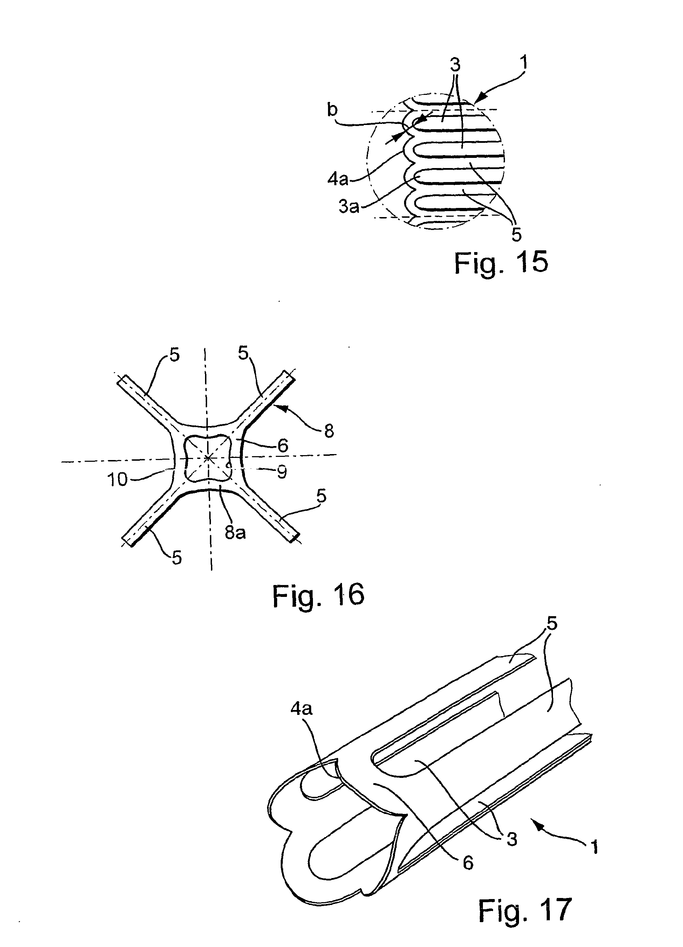

In den Fig. 15 bis 28 sind weitere vorteilhafte Realisierungen erfindungsgemäßer Drahtkorbeinheiten veranschaulicht. Dabei sind der Übersichtlichkeit halber für identische oder funktionell äquivalente Elemente jeweils gleiche Bezugszeichen verwendet.

Die Fig. 15 bis 17 zeigen eine Variante der Drahtkorbeinheit von Fig. 1, bei der statt des geradlinigen Rohrstirnendes 4 des Beispiels der Fig. 1 bis 14 das Rohrstirnende des Rohrstückrohlings mit einer wellenförmigen äußeren Profilierung 4a versehen ist, wie sie aus der Rohrabwicklung des vorderen Rohrendbereichs gemäß Fig. 15 und der Perspektivansicht des vorderen Rohrendbereichs gemäß Fig. 17 ersichtlich ist. Diese wellenförmige Profilierung 4a korrespondiert mit einem sich entsprechend stumpf verjüngenden Vorderabschluss 3a der rohrmantelteilenden Axialschlitze 3, so dass der Drahtverbindungsbereich 6 in diesem Beispiel über den gesamten Rohrumfang hinweg eine im Wesentlichen gleichbleibende axiale Breite b und einen wellenlinienförmigen Verlauf besitzt. Fig. 16 zeigt in Draufsicht das Vorderende der aus diesem modifiziert gestalteten Rohrstück nach Aufbiegen der Drahtstücke 5 erhaltenen Drahtkorbeinheit. Im Vergleich zum Ausführungsbeispiel der Fig. 1 bis 14, siehe insbesondere Fig. 13, ergibt sich für den Drahtverbindungsbereich 6 ein modifizierter Innenrandverlauf 9, der nicht wie in Fig. 13 praktisch kreisrund ist, sondern eher dem quadratischen Außenrandverlauf 10 des Drahtverbindungsbereichs 6 folgt.

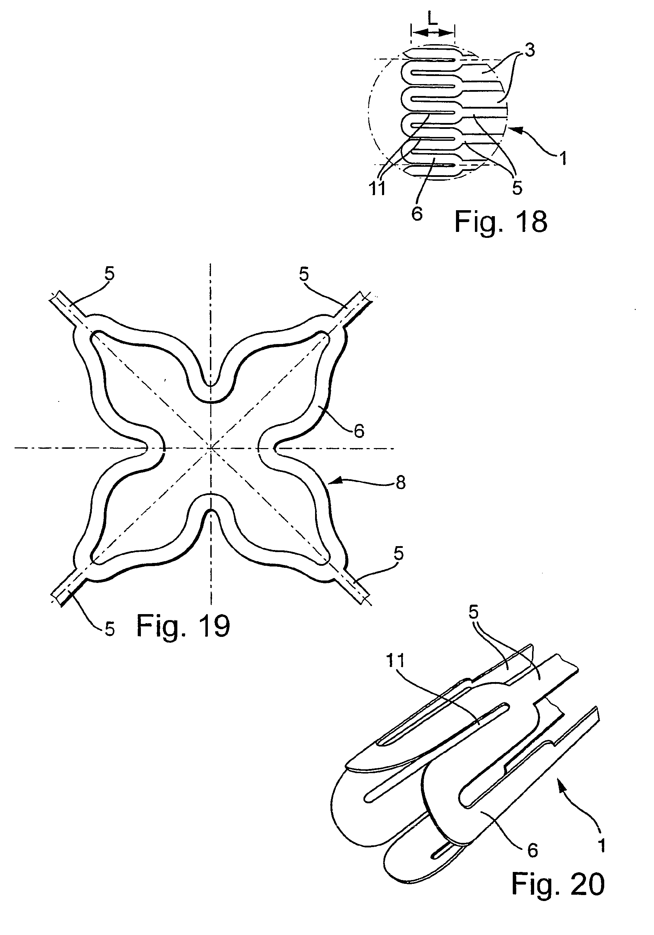

Die Fig. 18 bis 20 veranschaulichen ein Beispiel, bei dem von der vorderen Rohrstirnseite her ausgeprägte schlitzförmige axiale Ausnehmungen 11 in Umfangsrichtung alternierend zu den mit Abstand vor der Rohrstirnseite endenden Axialschlitzen 5 so eingebracht sind, dass sie über eine gewisse axiale Länge L mit den Axialschlitzen 5 überlappen. Im Überlappungsbereich L sind die Axialschlitze 5 verjüngt, d.h. sie weisen dort eine geringere Breite als im dahinter anschließenden Bereich auf. Die Axialschlitze 5 und die schlitzförmigen Ausnehmungen 11 weisen im Überlappungsbereich L jeweils eine im Wesentlichen gleichbleibende Breite auf. Fig. 19 zeigt wiederum in Draufsicht das Vorderende der zugehörigen, nach Aufbiegen der Drahtstücke 5 erhaltenen Drahtkorbeinheit. Durch die schlitzförmigen Ausnehmungen 11 ist in diesem Beispiel der Drahtverbindungsbereich 6 stärker gefaltet, und im Fall ansonsten gleicher Drahtkorbparameter ist der Drahtverbindungsbereich 6 nachgiebiger für das Aufbiegen der Drahtstücke 5 in die Drahtkorbgestalt als bei den zuvor beschriebenen Ausführungsbeispielen.

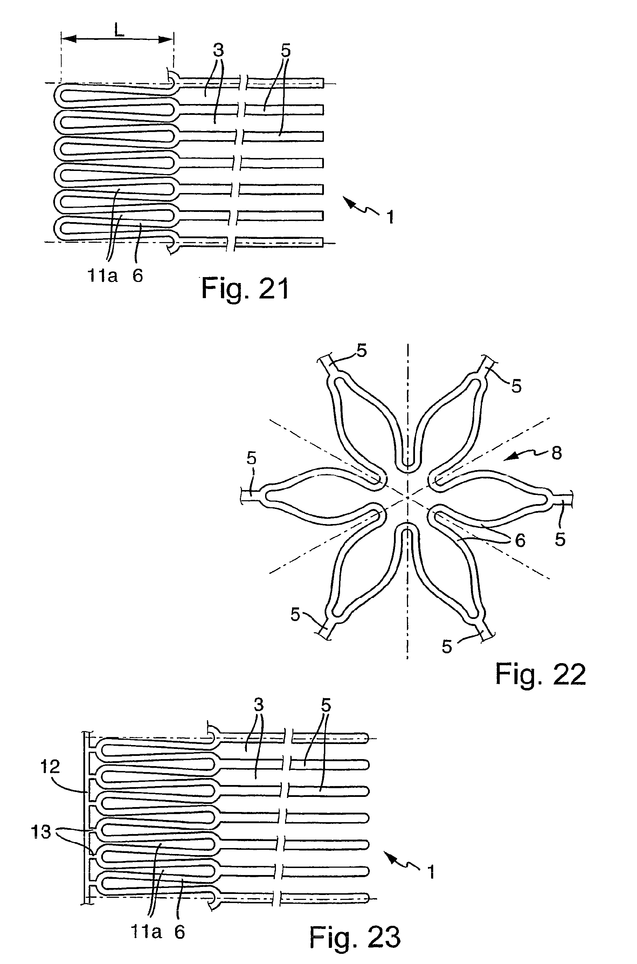

Die Fig. 21 und 22 veranschaulichen eine Variante, bei der die Drahtkorbeinheit aus sechs statt wie bei den vorherigen Beispielen aus vier Drahtstücken 5 gebildet ist, wozu entsprechend sechs statt vier Axialschlitze 3 in den Rohrstück-Rohling 1 eingebracht sind, wie aus der Rohrabwicklung von Fig. 21 ersichtlich. Des weiteren sind bei diesem Ausführungsbeispiel wie im Beispiel der Fig. 18 und 19 mit den Axialschlitzen 5 axial überlappende, schlitzförmige Ausnehmungen 11a von der Rohrstirnseite her alternierend zu den Axialschlitzen 5 eingebracht, d.h. in Umfangsrichtung mit zu den Winkellagen der Axialschlitze 5 alternierenden Winkellagen. Im Unterschied zum Ausführungsbeispiel der Fig. 18 bis 20 sind die schlitzförmigen Ausnehmungen 11a sich keilförmig in Richtung Rohrstirnende verjüngend ausgebildet. Dazu korrespondierend sind die Axialschlitze 5 in Richtung Rohrstirnseite sich keilförmig verbreiternd gestaltet. Diese Ausbildung der schlitzförmigen Ausnehmungen 11a und der Axialschlitze 5 im Überlappungsbereich L führt zu einer weiter erhöhten Faltungs-/Biegefähigkeit des Drahtverbindungsbereichs 6. Es ergibt dann nach Aufbiegen der Drahtstücke 5 eine Drahtkorbgestalt, deren Vorderende in der Draufsicht von Fig. 22 zu erkennen ist.

Fig. 23 zeigt eine Variante in Rohrabwicklungsdarstellung, die weitgehend dem Beispiel der Fig. 21 und 22 mit der Ausnahme entspricht, dass die keilförmigen axialen Schlitzausnehmungen 11a nicht bis zur Rohrstirnseite öffnend eingebracht sind, sondern am Rohrstirnende ein schmaler Rohrmantelteil 12 verbleibt, über den die einzelnen Faltungen 13 des Drahtverbindungsbereichs 6 vorderseitig miteinander verbunden bleiben.

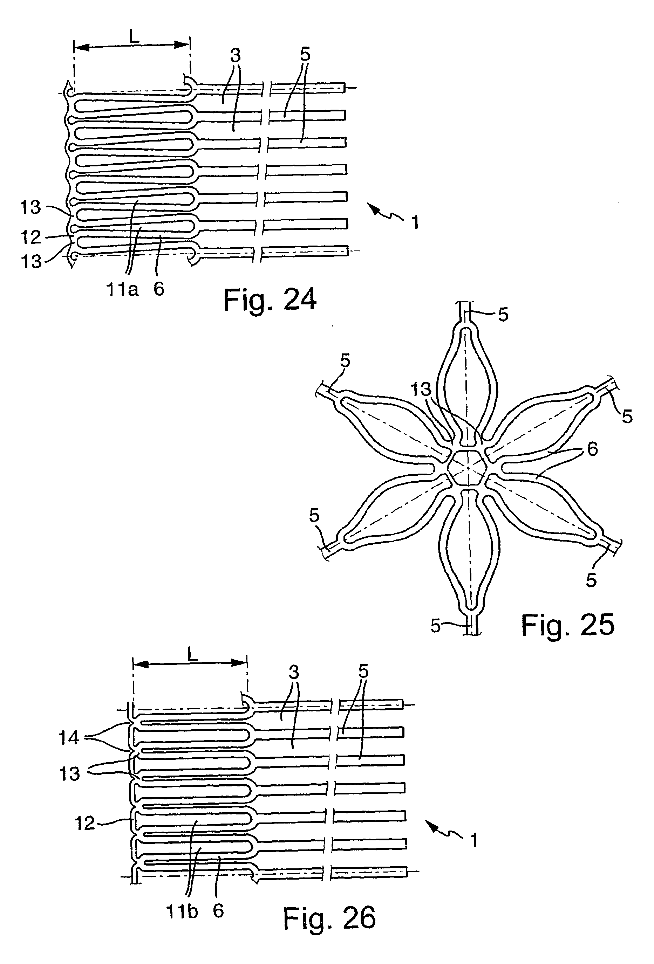

Die Fig. 24 und 25 zeigen ein zum Ausführungsbeispiel von Fig. 23 ähnliches Ausführungsbeispiel, wobei als Unterschied zusätzlich eine wellenförmige Profilierung der Rohrstirnseite vorgesehen ist. Dies hat zur Folge, dass der an der Rohrstirnseite umlaufend verbleibende Rohrmantelteil 12 entsprechend wellenlinienförmig verläuft. Fig. 25 zeigt wiederum in Draufsicht das Vorderende der aus dem solchermaßen vorgefertigten Rohrstück 1 nach Aufbiegen und Umformen der Drahtstücke 5 erhaltenen Drahtkorbeinheit.

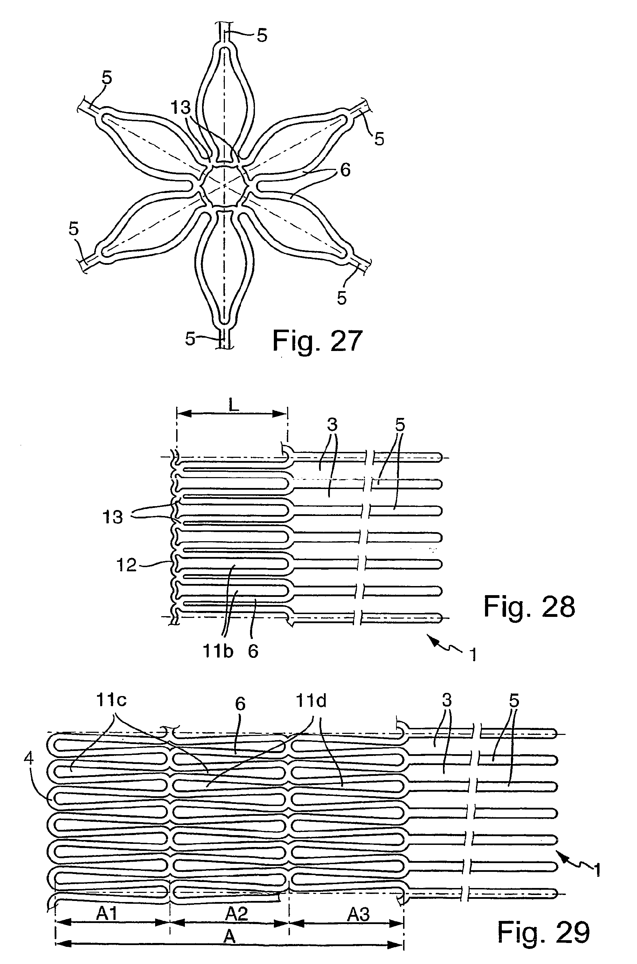

Die Fig. 26 und 27 veranschaulichen eine Variante ähnlich Fig. 23, wobei jedoch statt der keilschlitzförmigen Ausnehmungen 11a schlitzförmige Ausnehmungen 11 b konstanter Breite vorgesehen sind. Zudem ist in diesem Beispiel der vorderseitige umlaufende Rohrmantelteil 12 profiliert, indem er mit leichten Einkerbungen 14 jeweils im Verbindungsbereich einer Falte 13 des Drahtverbindungsbereichs 6 versehen ist. Dies führt zu dem in Fig. 27 gezeigten Aussehen des Vorderenden des daraus gebildeten Drahtkorbs.

Fig. 28 zeigt in der Rohrabwicklung eine Variante des Beispiels der Fig. 26 und 27, bei der die Profilierung des vorderseitig verbleibenden, umlaufenden Rohrmantelteils 12 dahingehend modifiziert ist, dass statt des im Beispiel der Fig. 26 und 27 geradlinigen Verlaufs die Abschnitte dieses Rohrmantelteils 12 zwischen den einzelnen Falten 13 des Drahtverbindungsbereichs gewellt verlaufen.

Fig. 29 zeigt in der Rohrabwicklung eine Variante des Beispiels der Fig. 21, bei der mehrere, axial versetzte Reihen von schlitzförmigen Ausnehmungen 11a, 11c, 11d innerhalb eines den Drahtverbindungsbereich 6 bildenden, vorderen axialen Rohrstückendabschnitts A vorgesehen sind. Speziell sind im gezeigten Beispiel zusätzlich zu der äußeren Reihe schlitzförmiger Ausnehmungen 11a, die zum Rohrstirnende 4 hin offen und in Umfangsrichtung aufeinanderfolgend angeordnet sind, zwei weitere Reihen von jeweils geschlossenen schlitzförmigen Ausnehmungen 11 c, 11 d in diesen vorderen Endbereich A des Rohrmantels eingebracht, wobei die geschlossenen schlitzförmigen Ausnehmungen 11 c, 11d in jeder der beiden Reihen wie die zum Rohrstirnende 4 hin offenen schlitzförmigen Ausnehmungen 11a in Umfangsrichtung mit jeweils gleichem Winkelabstand aufeinander folgen. Die schlitzförmigen Ausnehmungen 11 a, 11 c, 11 d sind in der aus Fig. 29 ersichtlichen Weise gestaltet, mit der Wirkung, dass der verbleibende Rohrmantel einstückig zusammenhängend bleibt und sich eine entsprechende Form für den Drahtverbindungsbereich 6 nach dem Aufbiegen der Drahtstücke 5 um einen gewünschten Aufbiegewinkel ergibt.

Wie aus Fig. 29 ersichtlich, besitzen die geschlossenen schlitzförmigen Ausnehmungen 11 c, 11 d der beiden entsprechenden Schlitzreihen jeweils eine Doppelkeilform aus zwei sich spiegelbildlich axial gegenüberliegenden, keilförmingen Ausnehmungen, wobei sich die beiden Reihen 11c, 11d in einem Axialbereich A2 mit je einer Keilhälfte in gegensätzlicher Keilform überlappen. Die axial äußere Reihe 11 c geschlossener schlitzförmiger Ausnehmungen überlappt mit einer Hälfte in einem äußeren Axialbereich A1 gegensätzlich mit den offenen keilförmigen Ausnehmungen 11 a. Die innere Reihe 11 d geschlossener schlitzförmiger Ausnehmungen überlappt mit ihren axial inneren Doppelkeilhälften in einem inneren Axialbereich A3 mit den gegensätzlich keilförmigen Enden der drahtstückbildenden Axialschlitze 3. Die geschlossenen schlitzförmigen Ausnehmungen 11 d der inneren Reihe alternieren in Umfangsrichtung mit den Axialschlitzen 3. Die geschlossenen schlitzförmigen Ausnehmungen 11c alternieren in Umfangsrichtung mit den weiter innen liegenden geschlossenen schlitzförmigen Ausnehmungen 11d und liegen folglich bei zu den Axialschlitzen 3 korrespondierenden Winkellagen. Die nach außen offenen schlitzförmigen Ausnehmungen 11a alternieren in Umfangsrichtung mit den axial überlappenden geschlossenen schlitzförmigen Ausnehmungen 11c und korrespondieren daher in ihrer Winkellage mit den axial inneren, geschlossenen schlitzförmigen Ausnehmungen 11 d.

Insgesamt führt diese Perforationsstruktur des Rohrmantelendbereichs im Beispiel von Fig. 29 zu einem entsprechend komplexen, jedoch noch immer einstückigen Drahtverbindungsbereich 6, wie er für bestimmte Anwendungsfälle besonders vorteilhaft ist. In analoger Weise sind weitere erfindungsgemäße Varianten mit beliebigen Strukturen schlitzförmiger Ausnehmungen realisierbar, die bei Bedarf auch mit merklicher Umfangsrichtungskomponente eingebracht sein können. Für bestimmte Anwendungsfälle kann auch wenigstens ein Teil der schlitzförmigen Ausnehmungen mit überwiegender Umfangsrichtungskomponente eingebracht sein. Wie aus den obigen Erläuterungen deutlich wird, bezeichnet der Begriff "Axialschlitz" vorliegend ausschließlich diejenigen Schlitzöffnungen im Rohrmantel, die zu dessen Aufteilung in die Drahtstücke dienen, während der Begriff "schlitzförmige Ausnehmung" speziell die Öffnungen im vorderen, den Drahtverbindungsbereich bildenden Endbereich des Rohrmantels bezeichnet.

Es versteht sich, dass erfindungsgemäß je nach Bedarf außer den gezeigten weitere Drahtkorbeinheiten realisierbar sind, die sich einstückig aus einem Rohrstück herstellen lassen. Die durch Aufteilen des Rohrmantels erzeugten Drahtstücke können in jede beliebige, gewünschte Korb- bzw. Ballonform gebogen werden. Dabei kann vorgesehen sein, dass die Drahtstücke in eine Aufnahmehülse oder dergleichen aufgenommen werden können und sich dadurch der Korbdurchmesser variabel einstellen lässt. Außer den gezeigten Beispielen mit vier bzw. sechs Drahtstücken sind selbstverständlich auch Drahtkorbeinheiten mit beliebiger anderer Anzahl von Drahtstücken möglich, indem der Rohrmantel des Rohrstück-Rohlings durch entsprechend viele Axialschlitze in die gewünschte Anzahl von Drahtstücken aufgeteilt wird. In gleicher Weise sind Breite, Länge, Biegewinkel und/oder Biegeform der einzelnen Drahtstücke beliebig wählbar. Dabei sind, wenn gewünscht, auch unterschiedliche Breiten und/oder unterschiedliche Winkelabstände der einzelnen Drahtstücke möglich. Zudem sind Drahtkorbrealisierungen möglich, bei denen eine Aufbiegung der Drahtstücke am Drahtverbindungsbereich um weniger als 90°, z.B. nur etwa 60°, oder um mehr als 90°, z.B. etwa 120°, zur Erzielung einer entsprechenden Drahtkorbform vorgesehen ist. Auch eine Umstülpung des aufgeteilten Rohrstücks ist möglich, bei der die hinteren Drahtstückenden um ca. 180° umgebogen sind, wobei die vorderen Drahtstückenden am Drahtverbindungsbereich z.B. um ca. 90° oder irgendeinen anderen Biegewinkel korbbildend aufgebogen sind.

Die Erfindung umfasst außer den gezeigten und oben beschriebenen Drahtkorbeinheiten auch andere Arten von Mehrdrahteinheiten, z.B. Drahtfiltereinheiten, wie sie ebenfalls in medizinischen Instrumenten eingesetzt werden. Zur Bildung eines solchen Drahtfilters werden die Drahtstücke nach Aufteilen des Rohrmantels in den gewünschten Filterfunktionszustand aufgebogen, z.B. um ca. 90°. Die hinteren, dem Drahtverbindungsbereich abgewandten Drahtstückenden bleiben dann nicht axial zusammengefasst, wie im Fall der Drahtkorbbildung, sondern werden auseinandergefächert in einer gewünschten Länge belassen. Durch die speziellen Gestaltungsmöglichkeiten des Drahtverbindungsbereichs, wie sie oben zur Drahtkorbbildung beschrieben sind und auch für die Drahtfilterbildung gelten, lässt sich beim Aufbiegen der Drahtstücke die ursprüngliche Rohröffnung durch die Verformung des Drahtverbindungsbereichs bei Bedarf in geeigneter Weise so verengen, dass eine gewünschte maximale Filterweite bereitgestellt wird, um Ablagerungen, Verklumpungen etc., die demgegenüber größere Abmessungen aufweisen, zurückhalten zu können. Auf diese Weise können z.B. plane oder trichterförmig gewölbte Drahtfiltereinheiten aus einem superelastischen Material realisiert werden. Je nach Anwendungsfall können zur Bildung entsprechender Drahtfilter auch bereits relativ geringe Aufbiegewinkel von deutlich weniger als 90° genügen, es kann aber auch, wie oben zur Drahtkorbbildung erwähnt, eine Umbiegung der Drahtstücke um mehr als 90° vorgesehen sein, um eine Drahtfiltereinheit entsprechender Form zu erzeugen. Des Weiteren kann die Struktur der schlitzförmigen Ausnehmungen im Drahtverbindungsbereich so gewählt sein, dass Querstegverbindungen des Rohrmantels verbleiben, die sich im Wesentlichen in Umfangsrichtung oder jedenfalls mit demgegenüber geringerer Axialkomponente erstrecken und zur Erzielung einer gewünschten Filternetzstruktur mit relativ gleichmäßiger Maschenweite beitragen können.

Während vorstehend vor allem auf die Anwendungsmöglichkeiten für medizinische Instrumente eingegangen wurde, versteht es sich, dass die erfindungsgemäße Mehrdrahteinheit für beliebige andere Anwendungen geeignet ist, die einen Drahtkorb bzw. Drahtkäfig, einen Drahtfilter oder dgl. benötigen.

## Patentansprüche

1. Mehrdrahteinheit, insbesondere für ein medizinisches Instrument, mit
- mehreren Drahtstücken (5), die einstückig aus einem Rohrstück (1) gebildet sind, dessen Rohrmantel (2) wenigstens in einem axialen Teilbereich durch mehrere, in Umfangsrichtung beabstandete Axialschlitze (3) in die Drahtstücke aufgeteilt ist, wobei die Drahtstücke an einem vorderen Endbereich (8a) miteinander verbunden bleiben, indem die Axialschlitze dort unter Belassung eines vorderen Drahtverbindungsbereichs (6) mit Abstand vor einem vorderen Rohrstirnende (4) enden, und in einem vorgebbaren Funktionszustand der Mehrdrahteinheit anschließend an den Drahtverbindungsbereich einen gebogenen Funktionszustand einnehmen,
**dadurch gekennzeichnet, dass**
- der vordere Drahtverbindungsbereich (6) im Funktionszustand der Mehrdrahteinheit zu einem im Wesentlichen spitzenlosen vorderen Endabschluss umgeformt ist, von dem aus sich die Drahtstücke (5) aufgebogen erstrecken, wozu die Axialschlitze (3) mit geeignet geringem axialem Abstand (a) vor dem vorderen Rohrstimende (4) enden.

2. Mehrdrahteinheit nach Anspruch 1, weiter **dadurch gekennzeichnet, dass** sie aus einem superelastischen Material gebildet ist.

3. Mehrdrahteinheit nach Anspruch 1 oder 2, weiter **dadurch gekennzeichnet, dass** die Drahtstücke (5) am Drahtverbindungsbereich (6) im Funktionszustand um mindestens etwa 60° zu einer Längsachse (7) des Rohrstücks auf- oder umgebogen sind und/oder der axiale Abstand (a) der Axialschlitze (3) vom vorderen Rohrstirnende (4) kleiner als ein Außendurchmesser (R) des Rohrstücks (1) oder kleiner als ein lnnendurchmesser (r) desselben oder im Wesentlichen gleich groß wie oder kleiner als eine Wanddicke (W) des Rohrstücks oder im Wesentlichen gleich groß wie oder kleiner als eine Breite (B) der Drahtstücke ist.

4. Mehrdrahteinheit nach einem der Ansprüche 1 bis 3, weiter **dadurch gekennzeichnet, dass** in den Rohrmantel (2) vom vorderen Rohrstirnende (4) her oder diesem benachbart eine oder mehrere schlitzförmige Ausnehmungen (11) eingebracht sind.

5. Mehrdrahteinheit nach Anspruch 4, weiter **dadurch gekennzeichnet, dass** sich die schlitzförmigen Ausnehmungen (11) axial bis in den Bereich zwischen die Axialschlitze (3) erstrecken.

6. Mehrdrahteinheit nach Anspruch 5, weiter **dadurch gekennzeichnet, dass** die Axialschlitze in ihrem axial mit den schlitzförmigen Ausnehmungen überlappenden Bereich verjüngt sind.

7. Mehrdrahteinheit nach Anspruch 6, weiter **dadurch gekennzeichnet, dass** die Axialschlitze (3) und die schlitzförmigen Ausnehmungen (11, 11 a, 11b) im überlappenden Axialbereich (L) mit im Wesentlichen gleichmäßiger Breite oder sich gegensätzlich keilförmig verjüngend ausgebildet sind.

8. Mehrdrahteinheit nach einem der Ansprüche 4 bis 7, weiter **dadurch gekennzeichnet, dass** mehrere, axial versetzte Reihen (11 a, 11 c, 11d) von in Rohrumfangsrichtung aufeinanderfolgenden schlitzförmigen Ausnehmungen in den Rohrmantel an einem vorderen Endbereich desselben eingebracht sind, wobei diese Reihen gegeneinander axial versetzt sind und sich in Axialrichtung überlappen oder nicht überlappen.

9. Mehrdrahteinheit nach einem der Ansprüche 1 bis 8, weiter **dadurch gekennzeichnet, dass** das Rohrstirnende mit einer äußeren, umlaufenden Profilierung (4a) versehen ist.

10. Mehrdrahteinheit nach einem der Ansprüche 1 bis 9, weiter **dadurch gekennzeichnet, dass** sie eine Drahtkorbeinheit mit einem korbbildenden Zustand der Drahtstücke als Funktionszustand oder eine Drahtfiltereinheit mit einem Filterzustand der Drahtstücke als Funktionszustand bildet.

11. Verfahren zur Herstellung einer Mehrdrahteinheit mit den Merkmalen eines der Ansprüche 1 bis 10, mit folgenden Schritten:
- Bereitstellen des Rohrstücks (1),
- Einbringen der Axialschlitze (3) in den Rohrmantel (2) zur Aufteilung in die Drahtstücke (5) und
- Biegen der Drahtstücke (5) und des Drahtverbindungsbereichs (6) in den Funktionszustand.

12. Verfahren nach Anspruch 11 zur Herstellung einer Mehrdrahteinheit mit den Merkmalen eines der Ansprüche 4 bis 10, weiter **dadurch gekennzeichnet, dass** vor dem Biegen der Drahtstücke (5) die schlitzförmigen Ausnehmungen (11, 11a, 11b) in den Rohrmantel eingebracht werden.

13. Verfahren nach Anspruch 11 oder 12, weiter **dadurch gekennzeichnet, dass** die Axialschlitze und/oder die schlitzförmigen Ausnehmungen durch Laserstrahlschneiden oder Wasserstrahlschneiden in den Rohrmantel eingebracht werden.

## Claims

1. Multiwire unit, preferably for a medical instrument, having
- a plurality of wire pieces (5) which are formed integrally from a tubular piece (1) whose tubular jacket (2) is divided into the wire pieces at least in an axial partial area by a plurality of axial slots (3) spaced in circumferential direction, where the wire pieces remain mutually linked at a forward end area (8a) in that the axial slots, while leaving a forward wire linkage area (6), end there at a distance from a forward tube front end (4), and where the wire pieces, in a predefinable functional state of the muliwire unit, assume a bent functional state in an area adjoining the wire linkage area,
**characterized in that**
- in the functional state of the multiwire unit, the forward wire linkage area (6) is deformed to an essentially tipless forward end termination, from which the wire pieces (5) extend in a bent-open manner, for the purpose of which the axial slots (3) end at an appropriately small axial distance (a) from the forward tube front end (4).

2. Multiwire unit according to Claim 1, further **characterized in that** it is formed of a superelastic material.

3. Multiwire unit according to Claim 1 or 2, further **characterized in that** the wire pieces (5) at the wire linkage area (6) in the functional state are bent open or bent over by at least approximately 60° with respect to a longitudinal axis (7) of the tubular piece, and/or the axial distance (a) of the axial slots (9) from the forward tube front end (4) is smaller than an outer diameter (R) of the tubular piece (1) or smaller than an inner diameter (r) thereof, or is essentially equally large as or smaller than a wall thickness (W) of the tubular piece, or essentially equally large or smaller than a width (B) of the wire pieces.

4. Multiwire unit according to any of Claims 1 to 3, further **characterized in that** one or more slot-shaped recesses (11) are provided in the tubular jacket (2) from the forward tube front end (4) or adjacent to the latter.

5. Multiwire unit according to Claim 4, further **characterized in that** the slot-shaped recesses (11) extend axially up to the area between the axial slots (3).

6. Multiwire unit according to Claim 5, further **characterized in that** the axial slots are tapered in their portion axially overlapping with the slot-shaped recesses.

7. Multiwire unit according to Claim 6, further **characterized in that**, in the overlapping axial area (L), the axial slots (3) and the slot-shaped recesses (11, 11a, 11b) are formed with an essentially uniform width or tapered in an oppositely wedge-shaped manner.

8. Multiwire unit according to any of Claims 4 to 7, further **characterized in that** a plurality of axially offset rows (11a, 11c, 11d) of slot-shaped recesses following one another in a tube circumferential direction are provided in the tubular jacket at a forward end area of the tubular jacket, said rows being axially mutually offset and overlapping or not overlapping with one another in the axial direction.

9. Multiwire unit according to any of Claims 1 to 8, further **characterized in that** the tube front end is provided with an outer, surrounding profiling (4a).

10. Multiwire unit according to any of Claims 1 to 9, further **characterized in that** it forms a wire basket unit with a basket-forming condition of the pieces as the functional state or a wire filter unit with a filtering condition of the wire pieces as a functional state.

11. Method of producing a multiwire unit having the characteristics of any of Claims 1 to 10, comprising the following steps:
- providing the tubular piece (1),
- forming the axial slots (3) in the tubular jacket (2) for division into the wire pieces (5), and
- bending the wire pieces (5) and the wire linkage area (6) into the functional state.

12. Method according to Claim 11 for producing a multiwire unit having the features of any of Claims 4 to 10, further **characterized in that** the slot-shaped recesses (11, 11a, 11b) are formed in the tubular jacket before the bending of the wire pieces (5).

13. Method according to Claim 11 or 12, further **characterized in that** the axial slots and/or the slot-shaped recesses are formed in the tubular jacket by laser beam cutting or water jet cutting.

## Revendications

1. Unité multifilaire, en particulier pour un instrument médical, comprenant :
- une pluralité de pièces en fil (5), formées d'une seul pièce à partir d'un morceau de tube (1), dont l'enveloppe de tube (2) est subdivisée, au moins dans une zone partielle axiale, en les pièces en fil, au moyen d'une pluralité de fentes axiales (3) espacées en direction périphérique, les pièces en fil étant reliées les unes aux autres sur une zone d'extrémité avant (8a), en ce que les fentes axiales s'achèvent à cet endroit en laissant subsister une zone de liaison de fil avant (6), à distance d'une extrémité frontale avant de tube (4) et, en un état fonctionnel, susceptible d'être prédéterminé, de l'unité multifilaire, prenant ensuite, à la zone de liaison de fils, un état fonctionnel plié,
**caractérisée en ce que**
- la zone de liaison de fil avant (6), à l'état fonctionnel de l'unité multifilaire, étant reformée en une terminaison d'extrémité pratiquement non pointue, à partir de laquelle les pièces en fil (5) s'étendent en étant ouvertes par pliage, ce pourquoi les fentes axiales (3) s'achèvent, avec une faible distance axiale (a) appropriée, avant l'extrémité frontale avant de tube (4).

2. Unité multifilaire selon la revendication 1, **caractérisée en outre en ce qu'**elle est formée en un matériau super-élastique.

3. Unité multifilaire selon la revendication 1 ou 2, **caractérisée en outre en ce que** les pièces en fil (5), à la zone de liaison de fil avant (6), à l'état fonctionnel, sont ouvertes par pliage ou repliées d'au moins à peu près 60° par rapport à un axe longitudinal (7) du morceau de tube, et/ou la distance axiale (a) des fentes axiales (3) vis-à-vis de l'extrémité frontale avant de tube (4) est inférieure à un diamètre extérieur (R) du morceau de tube (1), ou inférieure à un diamètre intérieur (r) de celui-ci ou sensiblement de valeur identique ou inférieure à une épaisseur de paroi (W) du morceau de tube, ou de valeur sensiblement égale ou inférieure à une largeur (B) des pièces en fil.

4. Unité multifilaire selon l'une quelconque des revendications 1 à 3, **caractérisée en ce qu'**un ou plusieurs évidements (11) en forme de fentes sont ménagés dans l'enveloppe de tube (2), en partant de l'extrémité frontale avant de tube (4) ou au voisinage de celle-ci.

5. Unité multifilaire selon la revendication 4, **caractérisée en outre en ce que** les évidements (11) en forme de fentes s'étendent axialement, jusque dans la zone située entre les fentes axiales (3).

6. Unité multifilaire selon la revendication 5, **caractérisée en outre en ce que** les fentes axiales sont effilées dans leur zone en chevauchement axial avec les évidements en forme de fentes.

7. Unité multifilaire selon la revendication 6, **caractérisée en outre en ce que** les fentes axiales (3) et les évidements (11, 11a, 11b) en forme de fentes, dans la zone axiale (L) en chevauchement, sont réalisées avec une largeur sensiblement régulière ou en s'effilant en forme de coin de manière opposée.

8. Unité multifilaire selon l'une des revendications 4 à 7, **caractérisée en outre en ce que** plusieurs rangées (11a, 11c, 11d), décalées axialement, d'évidements en forme de fentes se suivant dans la direction périphérique de tube, sont ménagées dans l'enveloppe de tube, sur une zone d'extrémité de celle-ci, ces rangées étant décalées axialement les unes par rapport aux autres et se chevauchant ou ne se chevauchant pas en direction axiale.

9. Unité multifilaire selon l'une des revendications 1 à 8, **caractérisée en outre en ce que** l'extrémité frontale de tube est munie d'un profilage de pourtour (4a) extérieur.

10. Unité multifilaire selon l'une des revendications 1 à 9, **caractérisée en outre en ce qu'**elle forme une unité à panier de fil, avec un état, formant le panier, des pièces en fil, en tant qu'état fonctionnel, ou une unité à filtre en fil, avec un état en filtre des pièces en fil en tant qu'état fonctionnel.

11. Procédé de fabrication d'une unité multifilaire présentant les caractéristiques d'une des revendications 1 à 10, avec les étapes suivantes :
- fourniture du morceau de tube (1),
- creusement des fentes axiales (3) dans l'enveloppe tubulaire (2), pour subdivision en les pièces en fil (5), et
- pliage des pièces en fil (5) et de la zone de liaison de fil (6), à l'état fonctionnel.

12. Procédé selon la revendication 11 de fabrication d'une unité multifilaire présentant les caractéristiques d'une des revendications 4 à 10, **caractérisée en outre en ce que** les évidements (11, 11a, 11b) en forme de fentes sont ménagés dans l'enveloppe de tube avant le pliage des pièces en fil (5).

13. Procédé selon la revendication 11 ou 12, **caractérisé en outre en ce que** les fentes axiales et/ou les évidements en forme de fentes sont réalisé(e)s par découpage au rayon laser, ou découpage au jet d'eau dans l'enveloppe de tube.
